# EUROPEAN PATENT APPLICATION

(11) **EP 2 062 865 A1**
(43) Date of publication of application: **27.05.2009**
(21) Application number: 07121337.5
(22) Date of filing: 22.11.2007
(51) Int. Cl.: C07C 11/18

(54) **Production of light olefins and isoprene from butane**

(71) Applicant: TOTAL PETROCHEMICALS RESEARCH FELUY, 7181 Seneffe (Feluy) (BE)
(72) Inventor: Vermeiren, Walter, 3530 Houthalen (BE); Bouvart, François, 60300 Senlis (FR)

(57) **Abstract**

Process for the selective production of ethylene, propylene and isoprene from light hydrocarbons comprising:
a) fractionating a butane fraction in a de-isobutanizer to obtain an enriched iso-butane fraction and an enriched normal-butane fraction,
b) cracking said normal-butane fraction and
optionally an ethane fraction,
optionally a propane fraction,
in a non-catalytic cracking zone to produce an olefin rich stream,
c) treating said olefin rich stream in a separating section to recover:
an ethylene stream,
a propylene stream,

d) transforming the recovered iso-butane of step a) into iso-butene or t-butyl hydroperoxide or partly into iso-butene and partly into t-butyl hydroperoxide,
e) optionally reacting iso-butene of step d), if any, with formaldehyde to make isoprene,
f) optionally reacting t-butyl hydroperoxide of step d), if any, with an olefin to give an epoxide and t-butanol and further separating t-butanol,
or optionally having t-butyl hydroperoxide of step d), if any, decomposed to t-butanol and reacted with formaldehyde to give isoprene,
or reacting a part of the t-butyl hydroperoxide of step d) with an olefin and having the remaining part decomposed to t-butanol and reacted with formaldehyde to give isoprene,
g) dehydrating the t-butanol recovered at step f), if any, into iso-butene and reacting said iso-butene with formaldehyde to make isoprene,
or reacting directly the t-butanol recovered at step f), if any, with formaldehyde to make isoprene,
or dehydrating the t-butanol recovered at step f), if any, into iso-butene, hydrogenating said iso-butene to iso-butane and oxidizing said iso-butane into t-butyl hydroperoxide, and recycling said t-butyl hydroperoxide,
or dehydrating the t-butanol recovered at step f), if any, into iso-butene, then disproportionating said iso-butene and propylene recovered at step c) (or 2-butene recovered at step c)), separating an isoamylene stream and converting the isoamylene into isoprene by dehydrogenation,
or making any combination of above routes of said step g),
h) optionally disproportionating iso-butene of step d), if any, and propylene recovered at step c) (or 2-butene recovered at step c)), separating an isoamylene stream and converting the isoamylene into isoprene by dehydrogenation,
at least one of steps e), f) and h) is not optional.

## Description

### [Field of the invention]

The present invention relates to a process for producing light olefins and isoprene from butane. By light olefins is meant ethylene and propylene. Olefins have long been desired as feedstocks for the petrochemical industries. Olefins such as ethylene, propylene and butenes are useful in preparing a wide variety of petrochemicals, including, but not limited to, polymers and isoprene. Isoprene is used as a basic chemical starting material for various chemical products and elastomers.

### [Background of the invention]

Methyl-tertiary-butyl-ether (MTBE) is being banned from the gasoline pool because of its negative impact on the environment in the actual refinery configurations. MTBE is conventionally produced by the reaction of isobutene with methanol over acidic resin catalysts. Isobutene can be obtained from existing refinery streams like the C4 cut produced on a fluid catalytic cracking unit or from steamcracking raw C4's. Aside of this isobutene that is obtained from byproducts, it can be produced on-purpose from field butanes that are for such extend dehydrogenated into isobutene and hydrogen. Typically, more than 30% of the world production of MTBE has been made from field butanes. Many of these units are condemned to close while no alternative exists for these dehydrogenation unit linked to MTBE production.

Conventionally isoprene is produced by extraction from pyrolysis gasoline, which is a byproduct of steamcracking of naphtha. The yield is typically very low, of the order of 1-3% of the produced ethylene. Hence it is difficult to justify this capital-intensive technology for only a small production capacity of isoprene. The process to isolate isoprene from pyrolysis gasoline consist first in the removal of cyclopentadiene by dimerisation and distillation. Next the pipirylenes are separated by superfractionation. The last steps consist in a extractive distillation using a solvent. Moreover the quality of isoprene obtained from pyrolysis gasoline is hard to guarantee as the specifications with respect to cyclopentadiene and pipirylenes are very severe and these compounds are plentiful present in the same pyrolysis gasoline. As pyrolysis gasoline contains only small amounts of isoprene (10-20%), a lot of byproducts (dicyclopentadiene and pipirylene) are produced according to the same laborious manner while their market value is not necessary in line with the evolution of the market value of isoprene.

Other routes to produce isoprene are the isolation of isoamylenes from refinery and petrochemical cuts and perform a dehydrogenation into isoprene. This process is typically done over iron oxide catalyst promoted with potassium compounds at temperatures above 600°C in presence of water steam and reduced pressure. As this reaction is limited by a thermodynamic equilibrium, only partial conversions can be obtained.

Isoprene can also be produced from isopentane by a double dehydrogenation.

In still another process, isoprene is produced by a two-step process. In the first step isobutene, tertiary-butanol, di-t-butyl ether, isobutanol, di-isobutyl ether, methyl-t-butyl ether or ethyl-t-butyl ether is condensed with two molecules of formaldehyde to form dimethyloxirane. The dimethyloxirane is separated and purified. In the second step the dimethyloxirane is decomposed under appropriate conditions into isoprene and one molecule of formaldehyde. An improvement on the latter two-step process is a one-step process, in which isobutene, tertiary-butanol, di-t-butyl ether, isobutanol, di-isobutyl ether, methyl-t-butyl ether or ethyl-t-butyl ether is directly reacted with formaldehyde into isoprene.

It has been discovered a flexible process to make ethylene, propylene and isoprene from butane and optionally ethane. In said process the butane fraction is treated in a de-isobutanizer to obtain an enriched iso-butane fraction and an enriched normal-butane fraction.

Then the enriched normal-butane fraction, optionally with the ethane fraction, is cracked in a non-catalytic cracking (steam cracking) zone to produce an olefin rich stream comprising ethylene and propylene. The n-butene contained in said olefin rich fraction can be optionally reacted with ethylene to increase the propylene production.

The enriched iso-butane fraction can be dehydrogenated to isobutene, then isobutene reacts with formaldehyde to give 4,4-dimethyl-m-dioxane which can be further decomposed to isoprene. The enriched iso-butane fraction also can be oxidized to t-butyl hydroperoxide, said t-butyl hydroperoxide reacts with an olefin (by way of example propylene) to give an epoxide (by way of example propylene oxide) and t-butanol. Then t-butanol can be dehydrated to isobutene or reacted with formaldehyde to give isoprene. Alternatively t-butyl hydroperoxide can be decomposed to t-butanol and reacted with formaldehyde to give isoprene.

In many countries there are hydrocarbon feedstocks comprised of ethane, propane and butane. Propane has a high value as LPG, therefore it is separated and sold as LPG and the remaining ethane and butane can be converted to ethylene, propylene and higher olefins. Steamcracking of n-butane produces ethylene and propylene, this is an advantage of the present invention over the usual steamcracking of ethane which does only produce ethylene. In the steamcracking of ethane the amount of propylene is often not sufficient to install the required equipment in order to recover the propylene.

Steamcracking of iso-butane results in low ethylene yields, high propylene yields and higher fuel gas make. Steamcracking of iso-butane results in significant liquid (C5+) products yield that accelerate coking of the furnace coils and require complicated oil quench equipment in order to minimise fouling downstream of the steamcracker furnace. This is an advantage of the present invention in which iso-butane is removed from butanes before steamcracking.

The steamcracking of n-butane produces butenes and metathesis (or disproportionation) reaction between ethylene and butene-2 allows increasing the propylene production of a steamcracker. Steamcracking of iso-butane results in high iso-butene make that is diluted in other hydrocarbons having 4 carbons. The presence of iso-butene has to be minimised in a metathesis reaction as iso-butene results in heavier hydrocarbons and hence loss of potential butene-2 that can make more propylene. This is again an advantage of the removal of iso-butane before steamcracking of butane.

The above process has not been described and not suggested in the prior art.

US 5,523,502 describes an integrated process for the selective production of olefins from hydrocarbons comprising:
(a) cracking a first hydrocarbon feedstock in a deep catalytic cracking process comprising contacting said heavy hydrocarbon feedstock with a solid, acidic catalyst, in a fluidized or moving bed or dense phase transfer line reactor, in the presence of steam to produce a first olefin-containing effluent;
(b) cracking a second hydrocarbon feedstock in a non-catalytic steam cracking process comprising thermally cracking said second hydrocarbon feedstock in the presence of steam in a radiant zone of a thermal cracking furnace to produce a second olefin- containing effluent;
(c) combining said first olefin-containing effluent with said second olefin-containing effluent to produce a mixed olefin-containing effluent for downstream processing;
(d) recycling a portion of said mixed olefin-containing effluent to the steam cracking step (b) as at least a portion of said second hydrocarbon feedstock; and
(e) recovering an effluent of selectively produced olefins from the mixed olefin-containing effluent from the downstream processing step.

In said process the first hydrocarbon feedstock is selected from the group consisting of crude oil, naphtha, distillate, vacuum gas oil, residual oil and mixtures thereof and the second hydrocarbon feedstock comprises a light hydrocarbon feedstock selected from the group consisting of gas oils, naphthas, butanes, propane, ethane and mixtures thereof.

US 2005 0107650 A1 relates to a method of producing propylene from a hydrocarbon feed stream involving the steamcracking of the hydrocarbon and then processing the ethylene that is obtained to produce the propylene. The invention is particularly applicable to a feed stream, which is, all or mostly, ethane. More precisely it relates to a method of producing propylene from ethane comprising the steps of:
a) steamcracking an ethane or primarily ethane feedstock thereby producing a cracking product containing ethylene, hydrogen, ethane, methane, acetylene and C3 and heavier hydrocarbons;
b) treating said cracking product in an ethylene plant recovery section including removing said hydrogen, methane and C3 and heavier hydrocarbons therefrom and converting said acetylene therein primarily to ethylene to thereby produce a treated cracking product containing primarily ethylene and ethane and including fractionating said treated cracking product in a C2 fractionator and obtaining an ethylene fraction and a bottom ethane fraction;
c) recycling said bottom ethane fraction to said steam cracking;
d) reacting by dimerization in a dimerization section a first portion of said ethylene fraction thereby producing a butene-rich stream;
e) reacting by metathesis in a metathesis section the butene in said butene-rich stream with a second portion of said ethylene fraction thereby producing a propylene-rich stream; and
f) separating product propylene from said propylene-rich stream.

US 5,026,936 describes a method for the production of propylene which comprises:
(a) cracking a C4 or higher olefin and/or paraffin hydrocarbon over a zeolite catalyst at conditions favoring production of ethylene and propylene to form a mixture comprised of ethylene, propylene and butene,
(b) separating ethylene and butene from the step (a) reaction mixture,
(c) metathesizing said separated ethylene and butene from the step (a) reaction mixture to form additional propylene, and
(d) recovering propylene formed in step (a) and step (c).
In the example of the above patent the C4 fraction to be cracked in step a) is a raffinate 2 comprising essentially n-butenes and 14 % of paraffins. A raffinate 1 is essentially a mixture of iso-butenes, n-butenes and paraffins. By selective reaction of iso-butene with methanol to make methyl-tertiary-butyl-ether (MTBE) raffinate 1 is converted to raffinate 2. Now for environmental considerations production of MTBE is decreasing sharply, thus raffinate 2 is less and less available.

US4091046 describes a process wherein isoamylene is produced from isobutane by cracking isobutane, separating a propylene stream and an isobutene stream from the cracked product, disproportionating the propylene and isobutene and separating an isoamylene stream from the disproportionated stream. The isoamylene can be converted into isoprene by dehydrogenation. The isobutane can be produced from normal butane by skeletal isomerization. The ethylene produced during the disproportionation can be dimerized and the butylenes obtained can be either reintroduced into the disproportionation reaction or can be dehydrogenated to butadiene. Isobutane removed from the cracked product, as well as isobutane removed from the disproportionated stream, is recycled into the cracking reaction. Hydrogen produced during the cracking reaction can be used either in the isomerization step for converting normal butane to isobutane, or can be used for hydrogenation of acetylenes produced during the conversion of the normal butene to butadiene. This process requires to isomerize butane.

### [Brief description of the invention]

The present invention relates to a process for the selective production of ethylene, propylene and isoprene from light hydrocarbons comprising:
a) fractionating a butane fraction in a de-isobutanizer to obtain an enriched iso-butane fraction and an enriched normal-butane fraction,
b) cracking said normal-butane fraction and
   optionally an ethane fraction,
   optionally a propane fraction,
   in a non-catalytic cracking zone to produce an olefin rich stream, preferably the ethane fraction, if any, the propane fraction, if any, and normal-butane fractions are cracked in separate non-catalytic cracking zones,
c) treating said olefin rich stream in a separating section to recover :
   an ethylene stream,
   a propylene stream,
d) transforming the recovered iso-butane of step a) into iso-butene or t-butyl hydroperoxide or partly into iso-butene and partly into t-butyl hydroperoxide,
e) optionally reacting iso-butene of step d), if any, with formaldehyde to make isoprene,
f) optionally reacting t-butyl hydroperoxide of step d), if any, with an olefin to give an epoxide and t-butanol and further separating t-butanol,
   or optionally having t-butyl hydroperoxide of step d), if any, decomposed to t-butanol and reacted with formaldehyde to give isoprene,
   or reacting a part of the t-butyl hydroperoxide of step d) with an olefin and having the remaining part decomposed to t-butanol and reacted with formaldehyde to give isoprene,
g) dehydrating the t-butanol recovered at step f), if any, into iso-butene and reacting said iso-butene with formaldehyde to make isoprene,
   or reacting directly the t-butanol recovered at step f), if any, with formaldehyde to make isoprene,
   or dehydrating the t-butanol recovered at step f), if any, into iso-butene, hydrogenating said iso-butene to iso-butane and oxidizing said iso-butane into t-butyl hydroperoxide, and recycling said t-butyl hydroperoxide,
   or dehydrating the t-butanol recovered at step f), if any, into iso-butene, then disproportionating said iso-butene and propylene recovered at step c) (or 2-butene recovered at step c)), separating an isoamylene stream and converting the isoamylene into isoprene by dehydrogenation,
   or making any combination of above routes of said step g),
h) optionally disproportionating iso-butene of step d), if any, and propylene recovered at step c) (or 2-butene recovered at step c)), separating an isoamylene stream and converting the isoamylene into isoprene by dehydrogenation,
   at least one of steps e), f) and h) is not optional.

It would not depart from the scope of the invention to provide a flexible process for the production of ethylene, propylene and isoprene from normal-butane and propane or from normal-butane, ethane and propane. This can happen when there is an overcapacity of propane on the LPG market. Steamcracking of propane produces high amounts of ethylene but rather low amounts of propylene; hence the fuel gas (mainly methane) make is high. Fuel gas make can be considered as loss of potential chemical value.

In an embodiment of the invention a normal-butane fraction and an ethane fraction are cracked in the non-catalytic cracking zone. Optionally the normal-butane fraction and the ethane fraction are cracked in two separate non-catalytic cracking zones.

In another embodiment of the invention a normal-butane fraction and a propane fraction are cracked in the non-catalytic cracking zone. Optionally the normal-butane fraction and the propane fraction are cracked in two separate non-catalytic cracking zones.

In another embodiment of the invention a normal-butane fraction, an ethane fraction and a propane fraction are cracked in the non-catalytic cracking zone. Optionally the normal-butane fraction, the ethane fraction and the propane fraction are cracked in two separate non-catalytic cracking zones. By way of example the normal-butane fraction and a part of the propane fraction are cracked in a non catalytic cracking zone and the ethane fraction and the remaining part of the propane fraction are cracked in a separate non catalytic cracking zone. Optionally the normal-butane fraction, the ethane fraction and the propane fraction are cracked in three separate non-catalytic cracking zones. This means the normal butane fraction is cracked in a non catalytic cracking zone, the ethane fraction is cracked in a separate non catalytic cracking zone and the propane fraction is cracked in a separate non catalytic cracking zone.

In another embodiment of the invention a light hydrocarbon feedstock comprising essentially ethane, propane and butane is fractionated to obtain a C3 fraction used as LPG, an ethane fraction and a butane fraction. The butane fraction is sent to the cracking zone through the de-isobutanizer, the ethane fraction is sent to the cracking zone.

Although, it is not the essence of the invention, propane can be substituted to butane in case butane would be temporarily in short supply. This would allow the petrochemical complex to continue to operate.

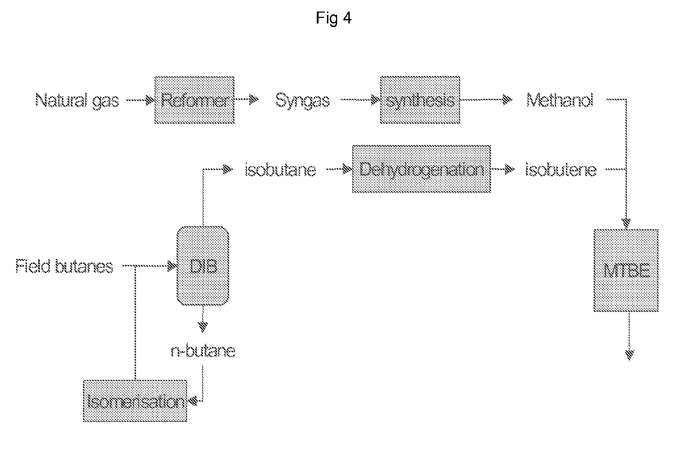

The present invention is of interest for retrofitting of an MTBE unit. Fig 4 illustrates a production of MTBE, DIB is the de-isobutanizer, "isomerisation" is the unit to convert n-butane to iso-butane, then iso-butane reacts with methanol to produce MTBE. Methanol is made from natural gas.

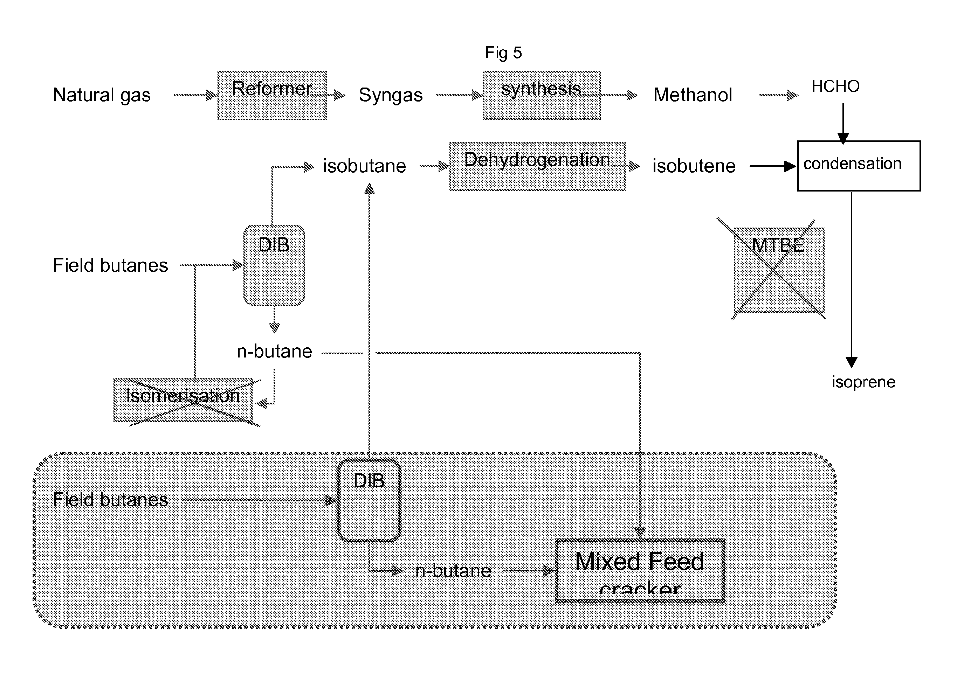

Fig 5 illustrates a process deriving from fig 4 wherein the n-butane isomerisation and the MTBE units are closed; n-butane originally sent to the isomerisation now is sent to a mixed feed cracker which is by way of example a steam cracker. An additionnal DIB is inserted to treat more butanes; n- butane is sent to said mixed feed cracker and the iso-butane is sent to the dehydrogenation to produce isobutene. Methanol is converted to HCHO and a condensation unit is inserted, in said condensation unit isoprene is made by reaction of HCHO with isoprene.

The present invention is a retrofitting of an iso-butane based MTBE unit to produce isoprene said iso-butane based MTBE process comprising :
1) sending a butane feedstock to a de-isobutanizer to recover an iso-butane overhead and a n-butane,
2) isomerizing the n-butane of step 1) in an isomerization unit and sending back the obtained iso-butane to the de-isobutanizer of step 1),
3) dehydrogenating the iso-butane of step 1) into isobutene,
4) reacting isobutene of step 3) with methanol to produce MTBE,
   Wherein,
5) the isomerization of step 2) and the MTBE reaction of step 4) are cancelled,
6) methanol is converted to HCHO and reacted with isobutene recovered at step 2) to produce isoprene,
7) n-butane recovered at step 1) is sent to a cracker, preferably a steam cracker.

In a specific embodiment is inserted an additionnal de-isobutanizer fed with a butane feedstock, the iso-butane recovered from said additionnal de-isobutanizer is sent to the dehydrogenation unit of step 3) and the n-butane recovered from said additionnal de-isobutanizer is sent to the cracker of step 7).

### [Detailed description of the invention]

**As regards steps a), b) and c)** they are known per se. Of course ethylene and propylene are the main olefins, but there are C4 olefins such as 1-butene and 2-butene.

As regards step c) in a specific embodiment it comprises :
treating said olefin rich stream of step b) in a separating section comprising:
   removing hydrogen and methane,
   recovering an ethylene stream,
   recovering an ethane stream and recycling said stream to the cracking zone,
   recovering a propylene stream,
   recovering a propane stream, optionally recycling said stream to the cracking zone,
   recovering a C4 stream,
   removing the heavies.

In another specific embodiment the above step c) is followed by a step c1) comprising :
selectively hydrogenating the dienes and alkynes in the C4 stream produced in step c) into their corresponding olefins.

In another specific embodiment above step c) and step c1) are followed by a step c2) comprising :
reacting by metathesis in a metathesis section the C4 stream produced in step d) and a part of the ethylene stream produced in step c) to produce propylene,

In another specific embodiment above step c), step c1) and step c2) are followed by a step c3) comprising :
treating the effluent of step c2) to recover :
   an ethylene stream optionally recycled to the metathesis section,
   a propylene stream,
   a C4 and heavies stream,

In another specific embodiment above step c), step c1), step c2) and step c3) are followed by a step c4) comprising :
treating the C4 and heavies stream produced in step c3) which is either hydrogenated, or not-hydrogenated and recycled to the cracking zone optionally through a de-isobutanizer, or recycled directly to the cracking zone.

**As regards step d)** dehydrogenation of iso-butane to iso-butene is known per se. Conversion of iso-butane into t-butyl hydroperoxide is known per se and has been described, by way of example, in US 4128587, US 5399777 and US 5475147, the content of which is incorporated by reference in the present application.

**As regards step e)** reacting iso-butene with formaldehyde to make isoprene is know per se. Isobutene reacts with formaldehyde to give 4,4-dimethyl-m-dioxane which decomposes to isoprene. Said route is described, by way of example, in GB 1370899 and US 3972955, the content of which is incorporated by reference in the present application. By way of example, EP 106323 and EP1614671, the content of which is incorporated by reference in the present application, describe a route in which iso-butene or t-butanol are reacted with formaldehyde in acidic aqueous medium to produce isoprene.

The operating conditions and the catalyst are optimised such that directly isoprene is produced. The process is catalysed by acid catalysts. The operating conditions are chosen such that the isoprene is removed as quickly as possible form the reaction mixture upon its formation. This is generally being done by vaporisation of the formed isoprene together with non-converted isobutylene and water vapour. These vapours are condensed and the isoprene is isolated from the remaining isobutylene and water. The isobutylene can be recycled back into the conversion reactor. The most suitable isobutyl-moiety is isobutylene as it requires the least amount of heat to be introduced to maintain the reactor temperature. Alcohols or ethers are at least partially decomposed in the presence of the acid catalyst, which requires a significant amount of reaction heat. The isolated isoprene is typically very pure after distillation as no other hydrocarbons with five carbons can be produced out of isobutylene and formaldehyde. Typical byproducts are oligomers of isoprene and formaldehyde that are easy to separate from isoprene. The catalyst may be any acid, homogeneous or hetereogeneous. It is preferred that the catalyst is a high boiling acid that remains in the aqueous phase of the reactor and does not vaporises with the isoprene out of the reactor vessel. Examples of liquid homogeneous catalysts are sulphuric acid, hydrosulfuric acid, phosphoric acid, monohydrophosphoric acid, dihydrophosphoric acid, boric acid, nitric acid, methanesulfonic acid, para-toluyl-sulfonic acid, heteropolyacids etc. Heterogeneous acids may also be use, among others sulfonated crosslinked divinylstyrene, sulfonated polyfluorohydrocarbons, sulfonated amorphous silica's, sulfonated mesoporous silica's, sulfonated zirconia's, supported heteropolyacids, zeolites etc.

**As regards step f)** the reaction of t-butyl hydroperoxide with propylene to give propylene oxide and t-butanol is known per se and has been described, by way of example, in US 4036905, US 5274138, US 5539131 and US 7223875, the content of which is incorporated by reference in the present application. The reaction of the second § of step f) is described, by way of example, in US 5399794 and US 4922035, the content of which is incorporated by reference in the present application.

**As regards step g)** the dehydration of the t-butanol into iso-butene is known per se and has been described, by way of example, in US 2005-0014985, the content of which is incorporated by reference in the present application. Hydrogenation of isobutene to isobutane is known per se.

**As regards step g) and step h)** and the disproportionation of iso-butene and propylene to make isoamylene, then separation of an isoamylene stream and convertion of the isoamylene into isoprene by dehydrogenation, it is known per se and has been described, by way of example, in US 4091146, the content of which is incorporated by reference in the present application.

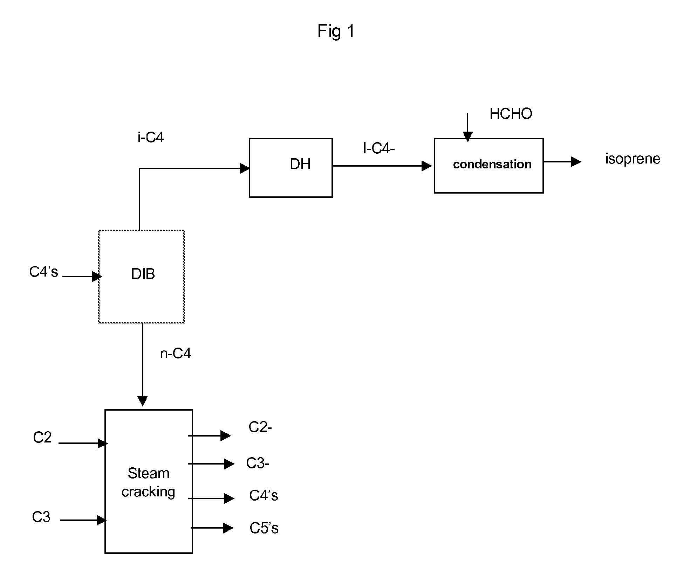

Various flow-shemes of the invention are hereunder described. Fig 1 illustrates an embodiment of the invention; DIB is the de-isobutanizer of step a) fed with butanes (C4's); n-butane (n-C4) is sent to the cracking zone to produce ethylene (C2-), propylene (C3-), C4 (C4's) and C5 (C4's). "Steam cracking" refers to steps b) and c). Iso-butane is sent to a dehydrogenation (DH) to produce isobutene (I-C4-); then isobutene is sent to "Condensation" wherein it is reacted with formaldehyde (HCHO) to make isoprene.

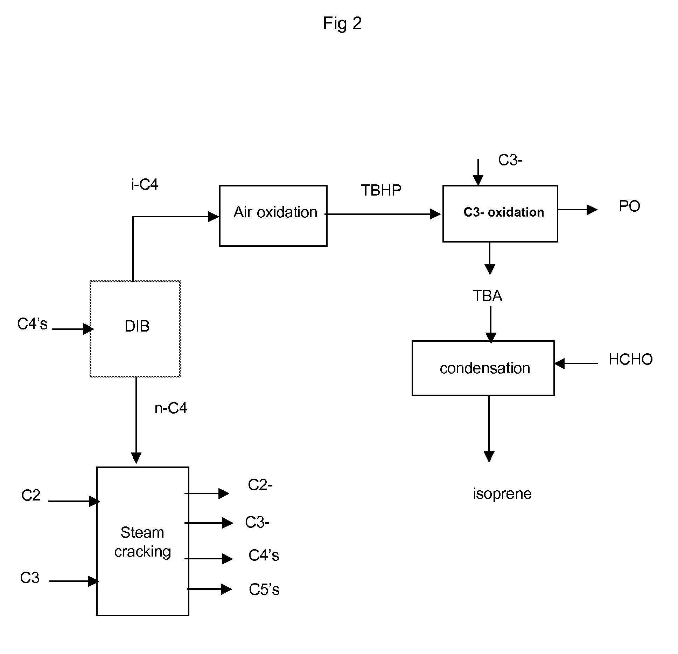

Fig 2 illustrates another embodiment in which the isobutene recovered at the DIB is sent to an air oxidation unit to make t-butyl hydroperoxide (TBHP). Said TBHP reacts with propylene (C3-) to produce t-butanol (TBA) and propylene oxide (PO). Then TBA reacts with formaldehyde to produce isoprene.

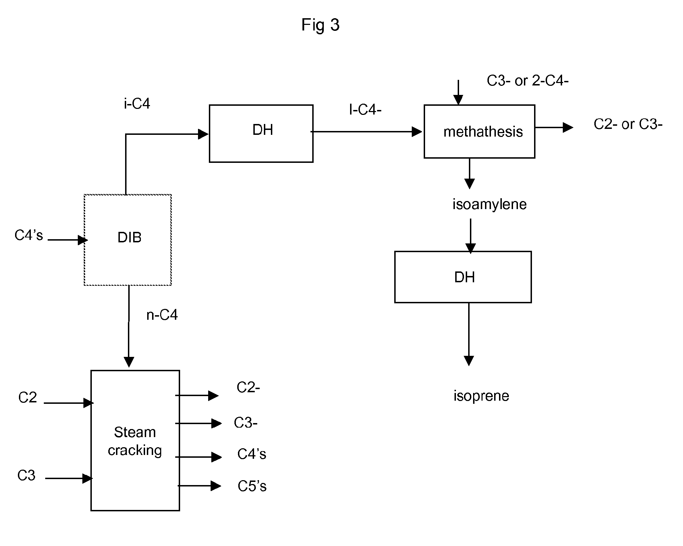

Fig 3 illustrates another embodiment in which the isobutene recovered at the DIB is sent to a dehydrogenation (DH) to produce isobutene (I-C4-); then isobutene is sent to the methathesis unit and reacted with propylen (C3-) or 2-butene (2-C4) to produce isoamylene which is in turn converted to isoprene by dehydrogenation in a DH unit.

## Claims

1. Process for the selective production of ethylene, propylene and isoprene from light hydrocarbons comprising:
a) fractionating a butane fraction in a de-isobutanizer to obtain an enriched iso-butane fraction and an enriched normal-butane fraction,
b) cracking said normal-butane fraction and
optionally an ethane fraction,
optionally a propane fraction,
in a non-catalytic cracking zone to produce an olefin rich stream,
c) treating said olefin rich stream in a separating section to recover :
an ethylene stream,
a propylene stream,
d) transforming the recovered iso-butane of step a) into iso-butene or t-butyl hydroperoxide or partly into iso-butene and partly into t-butyl hydroperoxide,
e) optionally reacting iso-butene of step d), if any, with formaldehyde to make isoprene,
f) optionally reacting t-butyl hydroperoxide of step d), if any, with an olefin to give an epoxide and t-butanol and further separating t-butanol,
or optionally having t-butyl hydroperoxide of step d), if any, decomposed to t-butanol and reacted with formaldehyde to give isoprene,
or reacting a part of the t-butyl hydroperoxide of step d) with an olefin and having the remaining part decomposed to t-butanol and reacted with formaldehyde to give isoprene,
g) dehydrating the t-butanol recovered at step f), if any, into iso-butene and reacting said iso-butene with formaldehyde to make isoprene,
or reacting directly the t-butanol recovered at step f), if any, with formaldehyde to make isoprene,
or dehydrating the t-butanol recovered at step f), if any, into iso-butene, hydrogenating said iso-butene to iso-butane and oxidizing said iso-butane into t-butyl hydroperoxide, and recycling said t-butyl hydroperoxide,
or dehydrating the t-butanol recovered at step f), if any, into iso-butene, then disproportionating said iso-butene and propylene recovered at step c) (or 2-butene recovered at step c)), separating an isoamylene stream and converting the isoamylene into isoprene by dehydrogenation,
or making any combination of above routes of said step g),
h) optionally disproportionating iso-butene of step d), if any, and propylene recovered at step c) (or 2-butene recovered at step c)), separating an isoamylene stream and converting the isoamylene into isoprene by dehydrogenation,
at least one of steps e), f) and h) is not optional.

2. Process acording to claim 1 wherein in step b) a normal-butane fraction and an ethane fraction are cracked in the non-catalytic cracking zone.

3. Process according to claim 2 wherein the normal-butane fraction and the ethane fraction are cracked in two separate non-catalytic cracking zones.

4. Process acording to claim 1 wherein in step b) a normal-butane fraction and a propane fraction are cracked in the non-catalytic cracking zone.

5. Process according to claim 4 wherein the normal-butane fraction and the propane fraction are cracked in two separate non-catalytic cracking zones.

6. Process according to claim 2 wherein in step b) a normal-butane fraction, an ethane fraction and a propane fraction are cracked in the non-catalytic cracking zone.

7. Process according to claim 6 wherein the normal-butane fraction, the ethane fraction and the propane fraction are cracked in three separate non-catalytic cracking zones.

8. Process according to claim 2 or claim 3 wherein :
a light hydrocarbon feedstock comprising essentially ethane, propane and butane is fractionated to obtain a C3 fraction used as LPG, an ethane fraction and a butane fraction,
the ethane fraction is sent to the cracking zone of step b),
the butane fraction is sent to the de-isobutanizer of step a) to obtain an enriched iso-butane fraction and an enriched normal-butane fraction,
said normal-butane fraction is sent to the cracking zone of step b).

9. Retrofitting of an iso-butane based MTBE unit to produce isoprene said iso-butane based MTBE process comprising :
1) sending a butane feedstock to a de-isobutanizer to recover an iso-butane overhead and a n-butane,
2) isomerizing the n-butane of step 1) in an isomerization unit and sending back the obtained iso-butane to the de-isobutanizer of step 1),
3) dehydrogenating the iso-butane of step 1) into isobutene,
4) reacting isobutene of step 3) with methanol to produce MTBE, Wherein,
5) the isomerization of step 2) and the MTBE reaction of step 4) are cancelled,
6) methanol is converted to HCHO and reacted with isobutene recovered at step 2) to produce isoprene,
7) n-butane recovered at step 1) is sent to a cracker, preferably a steam cracker.

10. Retrofitting of claim 9 wherein is inserted an additionnal de-isobutanizer fed with a butane feedstock, the iso-butane recovered from said additionnal de-isobutanizer is sent to the dehydrogenation unit of step 3) and the n-butane recovered from said additionnal de-isobutanizer is sent to the cracker of step 7).
